# EUROPEAN PATENT SPECIFICATION

(11) **EP 1 345 624 B1**
(45) Date of publication and mention of the grant of the patent: **21.06.2006**
(21) Application number: 01995704.2
(22) Date of filing: 20.12.2001
(51) Int. Cl.: A61K 39/39, A61P 37/00

(54) **INDUCTION OF TOLERANCE**
TOLERANZINDUKTION
INDUCTION DE TOLERANCE

(30) Priority: 22.12.2000 EP 00128438
(43) Date of publication of application: 24.09.2003
(73) Proprietor: Société des Produits Nestlé S.A., 1800 Vevey (CH)
(72) Inventor: TURINI, Marco, CH-1066 Epalinges (CH); GERMAN, Bruce, CH-1095 Lutry (CH); PECQUET, Sophie, CH-1012 Lausanne (CH)
(74) Representative: Thomas, Alain
(86) International application number: PCT/EP2001/015129
(87) International publication number: WO 2002/051437

(56) References cited:
- DATABASE BIOSIS [Online] BIOSCIENCES INFORMATION SERVICE, PHILADELPHIA, PA, US; 1982 LIFSCHITZ M D: "LLC-PK-1 CELLS DERIVED FROM PIG KIDNEYS HAVE A DEFECT IN CYCLO OXYGENASE" Database accession no. PREV198376063994 XP002170863 & JOURNAL OF BIOLOGICAL CHEMISTRY, vol. 257, no. 21, 1982, pages 12611-12615, ISSN: 0021-9258
- DATABASE BIOSIS [Online] BIOSCIENCES INFORMATION SERVICE, PHILADELPHIA, PA, US; 1992 CALDER P C ET AL: "THE INHIBITION OF T-LYMPHOCYTE PROLIFERATION BY FATTY ACIDS IS VIA AN EICOSANOID-INDEPENDENT MECHANISM" Database accession no. PREV199293088474 XP002170864 & IMMUNOLOGY, vol. 75, no. 1, 1992, pages 108-115, ISSN: 0019-2805
- DATABASE WPI Section Ch, Week 199711 Derwent Publications Ltd., London, GB; Class B05, AN 1997-115205 XP002170865 & JP 09 002958 A (SNOW BRAND MILK PROD CO LTD), 7 January 1997 (1997-01-07)
- GILBERT; WEIGLE JOURNAL OF IMMUNOLOGY vol. 151, no. 3, 01 August 1993, pages 1245 - 1254

## Description

The present invention pertains to a method for inducing tolerance to an antigenic material in an individual by administering a compound increasing the COX-2 (cyclo-oxygenase 2) activity in the individual's cells, in particular in cells involved in an immune reaction, and optionally also increasing the IFN-γ level, and a material or antigenic parts thereof, to which the individual develops adverse immune reactions. Specifically, the present invention pertains to a food or pharmaceutical composition containing such compounds and the antigenic material or parts thereof. In an alternative embodiment, the present invention also pertains to a novel ex vivo method for determining an individual's tendency to develop an allergic reaction to an particular material.

On of the modem diseases brought about by different environmental factors of the industrial society is allergy. Allergy is a hypersensitive state induced by an exaggerated immune response to a foreign agent affecting the lives of millions of living beings, often dictating what may be eaten, touched, smelled or even where living beings may live. The body's reaction to the foreign agent can range from minor inflammation and discomfort to even death in severe cases.

The clinical symptoms produced in the course of allergic reactions are a result of two different reactions, an early specific immune response and a late inflammatory reaction, respectively. Inhaled allergens, such as e.g. pollens or mite dust, mediate the early phase by stimulating high affinity immunoglobulin (IgE) receptors, e.g. mast cells and basophils, which in turn release histamine and cytokines. This early phase lasts for about 30 minutes. The cytokines released during the early phase from mast cells and basophils then mediate the late phase by recruiting inflammatory cells into the nasal and upper respiratory tract passages. The influx of eosinophils, macrophages, lymphocytes, neutrophils and platelets starts the inflammatory cycle. This late phase generally lasts for up to about 2 days and amplifies the initial immune response which in turn triggers the release of more inflammatory cells.

Current treatments of allergy generally focus on two generic approaches. One regimen merely treats the symptoms of allergy, wherein drugs, such as antihistamines, are utilized. One of the drawbacks of such methods resides in that this approach most often entails repeated doses of the corresponding drug to be administered and may involve undesirable side effects. In addition, it acts only to treat symptoms, not the underlying condition responsible for the hypersensitive condition. Another approach to treat allergy is the so-called desensitization therapy, which includes injection of specific allergens into an individual. Yet, before being able to start the treatment, the patient-specific allergens must first be recognized. Then the patient is injected repeatedly with low doses of the allergens. This approach involves discomfort and may require as many as 50 visits to a clinical practitioner. Moreover, the allergen to which the patient is hypersensitive cannot always be identified, making desensitization treatment impossible.

In addition to the drawbacks mentioned above neither of the above approaches guarantees elimination of the hypersensitive state. Likewise, neither can protect against the development of a hypersensitive state.

Apart from more and more people developing allergy to agents encountered in the environment, such as pollen, fabric etc. the number of consumers suffering from food allergy is steadily increasing. Particularly, serious problems are met with allergic reactions to cereals, nuts or milk since these products are consumed as the staple food in most countries and contain a high number of potential proteinaceous allergens.

For treating food allergy different approaches have been taken so far. One such approach aims at excluding the allergenic food material from the daily diet, which, however, proves difficult to accomplish in practice, since it requires strict diet revision, usually involving restrictive measures and may eventually affect the quality of life and/or inhibit the expected growth of young kids or newborns.

Another approach involves modifying the source of the allergenic material itself such that its allergic potential is reduced. This is generally achieved by depleting the food material of the allergenic component, which often raises problems in that the specific antigenic substance (allergen) in the food material is frequently not known so that in most cases it is not clear which component should be selectively removed. Another way to modify the allergenicity of the food material is to treat said food material in a specific way, such as by heat or by proteolytic enzymes.

In this respect US-P-4,293,571 discloses the preparation of a hypoallergenic composition, wherein a proteinaceous material is subjected to a hydrolysis treatment, the remaining, non hydrolysed proteins are subjected to coagulation by heat treatment followed by an ultrafiltration step to eliminate the coagulated material and macropeptides that might constitute allergens.

The problems encountered with food allergy are even more prominent in newborns, since they are not able to unambiguously communicate their discomfort to the attending person so that the allergic condition is most often detected only due to noticeable allergic symptoms, such as atopic dermatitis, respiratory and/or gastrointestinal troubles.

The prevention of the allergic material may include several constraints in the household and specific living rules for these infants. However, the key precaution in their diet is to avoid sensitization by native proteins. E.g. it has been shown that by drastically reducing the ingestion of native cow's milk proteins, the incidence of cow's milk allergy could be dramatically decreased.

Whatever the dietary option, the completion of immune tolerance to the milk proteins cannot be finally ensured without challenging the immune system with the blamed proteins. Until now, the tolerance status can only be checked by extrapolation, when infants do not manifest specific allergic response, after either local (Prick tests) or systemic challenge (DBPCFC).

Consequently, the problem of the present invention is to overcome the drawbacks of the prior art and to provide a novel means to treat or prevent allergic symptoms.

The problem has been solved by providing a method for inducing oral tolerance to a given antigenic food material in an individual, which comprises administering to an individual an antigenic food material and a substance raising the COX-2 (cyclo-oxygenase 2) activity in the individual's cells, in particular in cells involved in an immune reaction, such as lymphocytes, and/or increasing the IFN-γ (interferon γ) level.

In the figures,

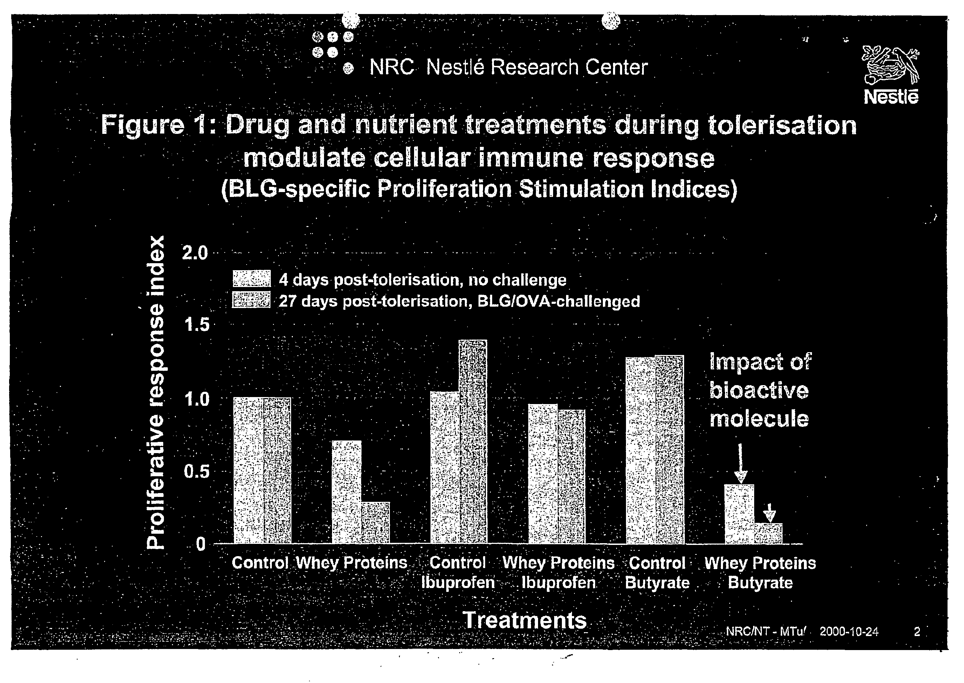

Fig. 1 shows the modulation of the cellular immune response by drug and nutrient treatments during tolerisation;
Fig. 2 shows a modulation of the humoral immune response to BLG by drug and nutrient treatments during tolerisation;
Fig. 3 shows measurements of PGE₂ and IFN-γ for assessing oral tolerance.

COX-2 is an enzyme catalyzing inter alia the synthesis of prostaglandins from arachidonic acid. Other known substrates for COX-2 include dihomo-gamma-linolenic acid (20:3n:6) and eicosapentaenoic acid (EPA, 20:5n-3) producing PGE₁ and PGE₃, respectively. The human COX-2 gene has been cloned and its genomic pattern and the responsiveness of its gene expression to different elements, such as cAMP, NF-κB and TGF-β, IL-1 or TNF-α has been described.

According to the present invention the term "increasing the COX-2 activity" is to be understood as increasing the enzymatic activity itself, such as by increasing the amount of cofactors or improving cooperation with the said cofactors, or by increasing transcription and/or translation of the protein in the cells or simply by increasing the amount of substrate.

Likewise, the term adverse immune reaction shall be understood as comprising any immune reaction detrimental to the individual, such as allergic reactions, auto-immune reactions and organ rejections.

In addition, the term cells of an individual shall comprise all cells, expressing COX-2 and which are involved in tolerogenic activities. These cells include in particular cells involved in immune reactions, such as antigen presenting cells, lymphocytes, in particular T-lymphocytes, neutrophils, granulocytes, monocytes etc.

The antigenic material to be given to an individual may be general food material itself. Examples for such food materials are milk, egg, soja, peanut, crustacean, fish, meat, sesame, whey, berries or apples.

The substance for increasing the COX-2 activity in the individual's cell, in particular in cells involved in an immune reaction, such as lymphocytes may be any substance capable of performing such an effect. Those substances may be easily identified by means of their effect on the synthesis of e.g. PGE₂ (prostaglandin E₂) from arachidonic acid in T-lymphocytes, which may be assayed by common enzymatic immunoassays.

According to a preferred embodiment such a substance, capable of effecting an increased activity of COX-2 in an individual's cells and optionally also an increased level of IFN-γ may be selected from the group comprising butyrate, n-6 polyunsaturated fatty acids (PUFAs) such as arachidonic acid or ceramides.

The antigenic material may be pretreated as desired. For instance, instead of including the entire material effecting an adverse immune reaction into the pharmaceutical composition the respective antigenic compound contained in the material, or parts thereof, such as e.g. particular allergenic peptides, may be isolated and these compounds may be included in the pharmaceutical composition.

The objective substance may also be included in a food material. In this case three alternatives are conceivable.

First, an antigenic material is included in a common food material together with the objective substance. This embodiment therefore contemplates addition of a known antigen, such as mentioned above in a food material, supplemented with an objective compound. As such food materials any liquid, such as water or even milk, jellies, sweets etc. may be proposed.

As a second alternative the food material may represent the antigenic material as such, that is, the food material inherently contains the potentially allergenic material. In this case, in order to arrive at a composition according to the present invention, the objective substance may simply be added to the food material. As such a food material any food to which an individual may develop an allergic reaction is embraced, including any kinds of berries, apples, peaches, etc.. In particular food products derived from cereals or cow's milk will represent a preferred kind of food, since these food products are known to contain highly allergenic compounds. Examples for such food products are yogurt, curd, cheese, fermented milks, milk based fermented products, ice cream or cereal products, such as fermented cereal based products, milk based powders, drinks, water-based jellies, infant formulae or even pet food.

The present invention, therefore, provides a novel and intriguing concept for inducing oral tolerance to a potentially allergenic food material, in an individual by simply including into the food material a substance capable of increasing COX-2 activity in the individual's cells, in particular in cells involved in immune reactions, such as lymphocytes, preferably T-cells, and optionally capable of increasing the IFN-γ level.

Therefore, the present invention teaches a method for inducing oral tolerance to a food material in an individual. This concept is particularly suitable in newborns which are about to develop tolerance or allergy to a food material. In applying the teachings of the present invention, an infant formula may be provided to which newborns won't develop an allergic reaction. What is more, the immune system of the newborns will rather be taught to tolerate said cow's milk proteins so that no problems will arise when feeding the newborns with the corresponding food material.

Alternatively, an antigenic component may be added to any other food material desired. For example, peanuts or the relevant allergenic components thereof may be added to fermented milk products, such as a yogurt in order to provide a yogurt with peanut taste and, by including the objective substance, the desired regimen. This applies likewise to the addition of pieces of berries, apples etc to such fermented milk products.

As will be appreciated the compositions of the present invention are perfectly suited to combat the development of allergy in an individual before it may arise. Without wishing to be bound to any theory it is believed that the concurrent exposure of the immune system to the antigen together with inducing a biological step involved in suppressing an exceeding immune reaction to said material teaches the immune system in general not to adversally react to the specific antigen presented. Thus, the concept underlying the present invention basically resides in modulating the biochemistry/metabolism of an individual's cells implicated in the oral tolerance induction, preferably of cells involved in an immune reaction.

According to the present invention it is therefore possible not only to prevent the onset of allergy but also to treat an already existing allergic condition, by "teaching" the immune system not to react to a material, that is concurrently presented with a substance capable of increasing COX-2 activity in cells of an individual, preferably cells involved in an immune reaction, and optionally increasing the IFN-γ level.

It will be understood that the amount of the objective substance in the food composition will be sufficient to yield the desired effect, that is to effect an increase in activity of COX-2 in the respective cells of the individual, preferably in lymphocytes, more preferably in T-lymphocytes, and optionally effecting an increase in the IFN-γ level in peripheral tissues, such that the individual's immune system will effectively be taught to tolerate the antigenic material once recognized as a foreign antigen to which a surpassing immune response is elicited.

The following examples will illustrate the invention in more detail, without limiting it thereto.

The following materials were used and methods applied:

### Animals

Female Balb/c mice, obtained from IFFA-Crédo (L'Abresle, France), were used for all experiments performed on three week old mice. All mice were bred and raised on a cow's milk free diet. For all experiments, mice were weighed on the day they were fed, in order to determine the gavage amount according to their body weight.

### Antigens

BLG (bovein lactoglobuline; 3 x crystallised) and OVA (ovalbumin; Grade V) were obtained from Sigma Chemical Co.. Whey protein concentrate (Lacprodan 80) was obtained from Danmark Protein AS (MD-Foods). It was produced by ultrafiltration of acid whey, and the protein content was 80%.

### Statistics

The following examples will illustrate the invention in more detail, without limiting it thereto.

The following materials were used and methods applied:

### Animals

Female Balb/c mice, obtained from IFFA-Crédo (L'Abresle, France), were used for all experiments performed on three week old mice. All mice were bred and raised on a cow's milk free diet. For all experiments, mice were weighed on the day they were fed, in order to determine the gavage amount according to their body weight.

### Antigens

BLG (bovein lactoglobuline; 3 x crystallised) and OVA (ovalbumin; Grade V) were obtained from Sigma Chemical Co.. Whey protein concentrate (Lacprodan 80) was obtained from Danmark Protein AS (MD-Foods). It was produced by ultrafiltration of acid whey, and the protein content was 80%.

### Statistics

Seric IgE responses were compared using ANOVA single factor tests. Significance was reached with p < 0.05.

### Example 1

### Treatment of mice

To start the experiment the mice were forced fed and/or injected i.p. with butyrate (which promotes COX-2 activity), ibuprofen (which inhibits COX activity) or saline (control) daily from day -3 to day 4. On day 0, the mice were fed by gastric feeding whey proteins (3mg/g of body weight) or same volume of saline water. Gavage products were dissolved in 0.3 ml of saline water. At day 4 of each experiment, all mice were immunised by intra-peritoneal injection of 0.08 mg of BLG as well as 0.08 mg of OVA in 0.04 ml sterile saline mixed with 0.16 ml 2 % Al(OH)₃ (Superfos Biosecotr A/S, Denmark).

At day 4 or at day 26, blood samples were obtained from cardiac aortic punction under 3 % Isoflurane anaesthesia (Abbott SA). Immediately after death, obtained by cervical dislocation, spleens were removed and pooled according to group of treatment in 20 ml chilled RPMI supplemented with 5% FCS. Spleen cell solutions were homogenised through a cellular sieve (Falcon) and purified from red cells using Histopaque density centrifugation (Sigma) at 390 x g for 20 min. Cells were co-cultured in.96-well flat-bottom microtitre plates (Costar), for 48 hours at a concentration of 5 x 10⁶ cells/ml in RPMI 1640 (Bioconcept) supplemented with 2 mM L-Glutamine (Seromed), 100 U penicillin/100 mg streptomycin (Seromed), 10 % FCS (Bioconcept) at 37°C in 5% CO₂, in the presence of 5, 2.5, 0.5, or 0.1 mg/mL of BLG (Sigma) or 250, 50, 10, 2 µg/mL of phytohaemagglutinin A (Seromed). (³H)Tdr (Amersham, Zurich) was added for the final 6 hours of culture and the plates were harvested and analysed by scintillation counting (TopCount, Canberra Packard, Zurich). (³H)Tdr incorporation results were expressed in cpm, as a mean of triplicate cultures, the blank-subtracted mean was then plotted against BLG concentration. Stimulation indices were calculated as the ratio of blank-subtracted test and control values at 2.5 mg BLG /mL and 50 µg PHA/mL.

Specific proliferation assays were performed for each group. Isolated serum were rapidly frozen at -20°C, until assayed for IgE specific for BLG.

### Example 2

### Measurement of specific IgE antibody by ELISA

Serum samples were assayed in duplicate for anti-BLG IgE by ELISA. Briefly, microtitre plates (NUNC Immunoplate Maxisorp F96, Denmark) were coated with 100 µl per well of BLG 0.5 mg/mL diluted in carbonate-bicarbonate buffer, pH 9.6, overnight at 4°C. Four washing steps with PBS-Tw (Phosphate-Buffered Saline-Tween 0.05%) were performed before each successive reagent addition. Unoccupied sites on the plate were blocked by 200 µL per well of 0.5% fish gelatin (Sigma) in PBS-Tw for 1 hour at RT. Serial dilutions of samples (three fold for serum) from individual mice were assayed in duplicate. Sera were first diluted 1/20 in PBS-Tw before the assay. After addition of sample dilutions and incubation for 1 hour at room temperature, a rat anti-mouse IgE peroxydase labelled antibody (Harlan Sera-Lab, USA) was added for 2 hours, followed by the OPD subtrate (Sigma). Optical densities were measured at 492 nm, after 15 mn of incubation at room temperature, using a Dynatech MR500 ELISA reader (Dynatech, Embrach-Embraport, Switzerland. Pooled samples from twenty non immunized female mice were used as negative controls in each plate. The cut-off dilutions were determined by calculating the dilution of the samples which gave twice the absorbance of the negative controls. Titers were expressed as the log₁₀ of the reciprocal of the cut-off dilution.

### Example 3

### Cytokine ELISA

Cytokine secretions were induced in 96-well microtitre plates (Costar), where 5 x 10⁵ spleen cells were stimulated with 2.5 mg/mL BLG per well. Cell culture supernatants were collected after a 48 hour stimulation. IFN-γ levels were measured using cytokine specific ELISA kit Endogen; which were performed according to the manufacturers' instructions. All cytokine samples were assayed in duplicate.

### Example 4

### Prostaglandin E₂ and interferon-gamma measurement

PGE₂ levels in the culture media, from lymphocytes cultured for 6 hrs or 12hrs, were determined by using an enzyme immunoassay (EIA) kit (Cayman Chemical,). Triplicate samples were assayed each at two dilutions.

Inducing tolerance by feeding mice with Whey proteins resulted in an increased PGE₂ and INF-γ production by BLG-stimulated lymphocytes in culture as compared to non-tolerised controlled mice, 5 days after tolerisation.

This production of PGE₂ and INF-γ is not observed when mice are treated with ibuprofen, an inhibitor of COX. On the other hand, mice treated with butyrate exhibit a further increased production of both PGE₂ and INF-γ compared to the positive control - Whey proteins fed mice.

### Example 5

### Proliferation

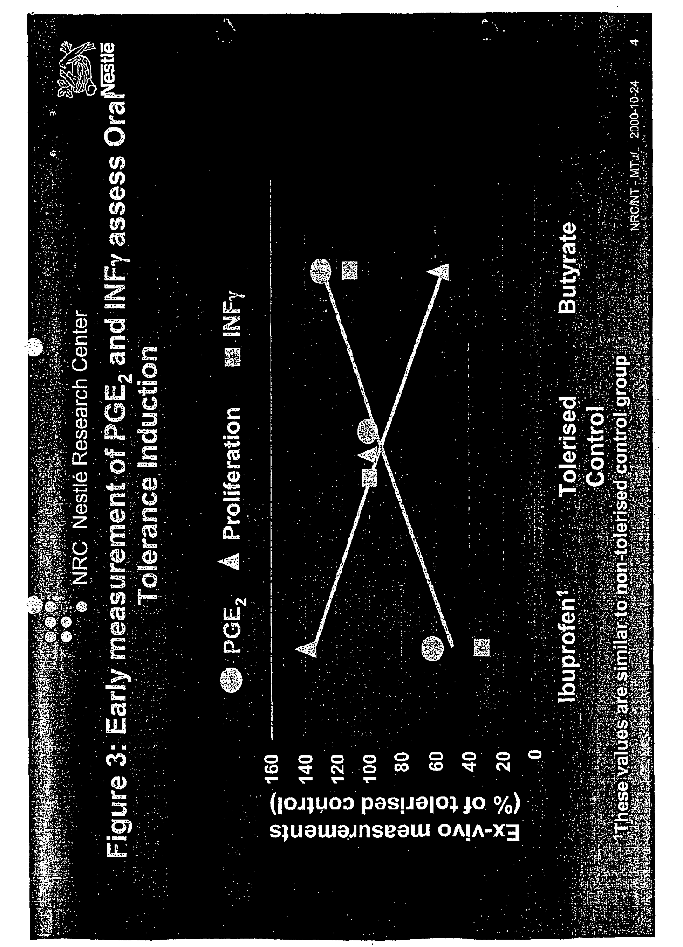

Inducing tolerance by feeding mice with Whey proteins results in a decreased proliferative response index, 4 days post-tolerisation (Figure 3). A stimulation index below 0.5 indicates that mice have been tolerised. This decreased of proliferative response index is not observed when mice are treated with ibuprofen, an inhibitor of COX. On the other hand, mice treated with butyrate exhibited a further decreased of proliferative response index as compared to the positive control - Whey proteins fed mice.

Similar and stronger results were observed at day 26 post-tolerisation in mice that had been challenged with BLG 4 days after feeding the tolerogen.

### Example 6

### IgE

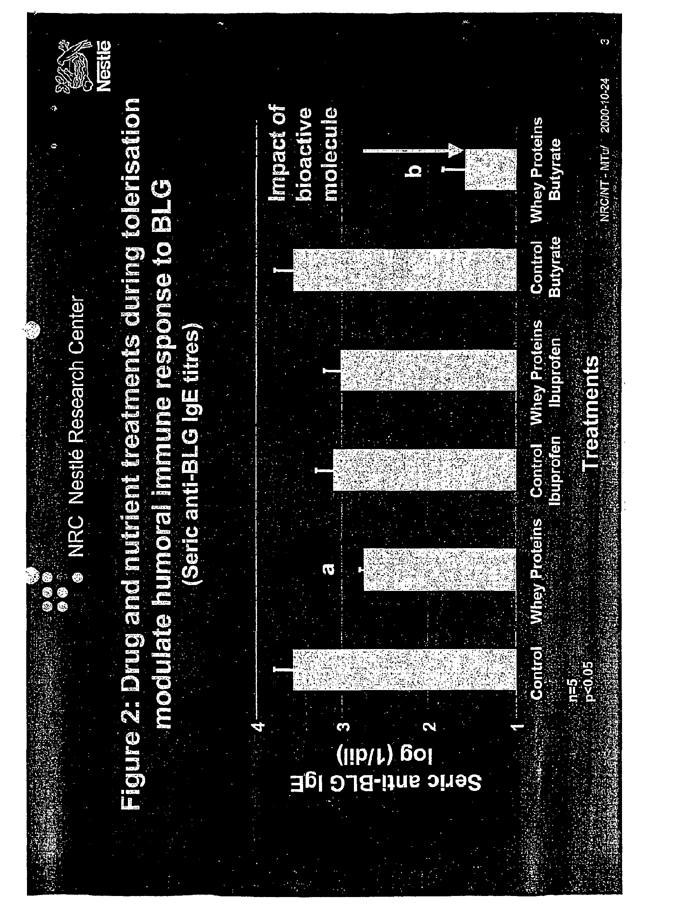

Inducing tolerance by feeding mice with Whey proteins resulted in a decreased of humoral anti-BLG IgE titres, 26 days post-tolerisation. This decrease was statistically significant (p<0.05) as compared to non-tolerised mice. This decreased humoral anti-BLG IgE titres is not observed when mice are treated with ibuprofen, an inhibitor of COX (see fig. 2).

On the other hand, mice treated with butyrate exhibit a further decreased of humoral anti-BLG IgE titres compared to the positive control - Whey proteins fed mice. This further decrease in humoral anti-BLG IgE titres was statistically significant (p<0.05) compared to tolerised mice (positive control)

## Claims

1. Use of an antigenic food material or antigenic parts thereof, and at least one substance increasing the activity of COX-2 (cyclo-oygenase 2) in cells of the individual and/or increasing the level of IFN-γ (interferon γ) in the manufacture of a composition for inducing oral tolerance to the antigenic food material.

2. The use of claim 1, wherein the food material is derived from milk, egg, soja, nuts, crustacean, fish, meat, sesame, whey, berries or apples.

3. The use of any of the preceding claims, wherein the at least one substance, capable of increasing the COX-2 activity in cells of the individual and optionally the level of IFN-γ is selected from the group consisting of butyrate, n-6 PUFA (n-6 polyunsaturated fatty acids), or ceramides.

## Patentansprüche

1. Verwendung eines antigenen Lebensmittelmaterials, oder von antigenen Teilen davon, sowie von wenigstens einer Substanz, die in Zellen der Person die Aktivität von COX-2 (Cyclooxygenase 2) erhöht und/oder den Spiegel an IFN-γ (Interferon-γ) erhöht, bei der Herstellung einer Zusammensetzung zur Induzierung einer oralen Toleranz gegenüber dem antigenen Lebensmittelmaterial.

2. Verwendung nach Anspruch 1, wobei das Lebensmittelmaterial sich von Milch, Ei, Soja, Nüssen, Krustentieren, Fisch, Fleisch, Sesam, Molke, Beeren oder Äpfeln ableitet.

3. Verwendung nach irgendeinem der vorausgehenden Ansprüche, wobei die wenigstens eine Substanz, die in der Lage ist, die COX-2 Aktivität in Zellen des Individuums sowie ggf. den Spiegel an IFN-γ zu erhöhen, ausgewählt ist aus der Gruppe, die besteht aus Butyrat, n-6 PUFA (n-6 polyungesättigten Fettsäuren) oder Ceramiden.

## Revendications

1. Utilisation d'un matériau alimentaire antigénique ou de parties antigéniques de celui-ci, et d'au moins une substance augmentant l'activité de la COX-2 (cyclo-oxygénase 2) dans des cellules de l'individu et/ou augmentant le taux d'IFN-γ (interféron γ) dans la fabrication d'une composition pour induire une tolérance orale au matériau alimentaire antigénique.

2. Utilisation selon la revendication 1, dans laquelle le matériau alimentaire est dérivé de lait, d'oeuf, de soja, de noix, de crustacé, de poisson, de viande, de sésame, de petit-lait, de baies ou de pommes.

3. Utilisation selon l'une quelconque des revendications précédentes, dans laquelle la au moins une substance, capable d'augmenter l'activité de la COX-2 dans des cellules de l'individu et, éventuellement, le taux d'IFN-γ, est choisie dans le groupe constitué par le butyrate, les AGPI n-6 (acides gras polyinsaturés du groupe n-6), ou les céramides.
